# FASCICULE DE BREVET EUROPEEN

(11) **EP 0 700 984 B1**
(45) Date de publication et mention de la délivrance du brevet: **25.11.1998**
(21) Numéro de dépôt: 95401920.4
(22) Date de dépôt: 21.08.1995
(51) Int. Cl.: C10G 70/02, C07C 7/167

(54) **Hydrogénation sélective de coupes hydrocarbonées renfermant des hydrocarbures monoinsaturés et polyinsaturés**
Selektive Hydrierung von einfach und mehrfach ungesättigten Kohlenwasserstoffen enthaltende Fraktionen
Selective hydrogenation of hydrocarbon fractions containing mono- and poly-unsaturated hydrocarbons

(30) Priorité: 08.09.1994 FR 9410943
(43) Date de publication de la demande: 13.03.1996
(73) Titulaire: INSTITUT FRANCAIS DU PETROLE, 92502 Rueil-Malmaison (FR)
(72) Inventeur: Euzen, Jean-Paul, F-69570 Dardilly (FR); Leger, Gérard, F-69300 Caluire (FR); Trambouze, Pierre, F-69300 Caluire (FR); Delhomme, Henri, F-69110 Sainte Foy Les Lyons (FR)

(56) Documents cités:
- EP-A- 0 212 202
- EP-A- 0 554 151
- GB-A- 866 395
- US-A- 3 492 220
- US-A- 3 493 492

## Description

La présente invention concerne le domaine de l'hydrogénation sélective de composés polyinsaturés contenus dans des coupes d'hydrocarbures ayant 2 à 20 atomes de carbone, de préférence 2 à 10, et avantageusement 2 à 5, et renfermant des hydrocarbures oléfiniques monoinsaturés et/ou des aromatiques. C'est en particulier le cas des essences provenant des procédés de craquage thermique et craquage catalytique.

Les coupes d'hydrocarbures dénommées par les hommes du métier coupes C₂, C₃, C₄ et C₅ peuvent provenir de divers procédés de conversion d'hydrocarbures par exemple du vapocraquage, de la viscoréduction, de la cokéfaction ou du craquage catalytique. Ces coupes contiennent ainsi assez souvent des quantités plus ou moins importantes d'hydrocarbures polyinsaturés tels que des hydrocarbures acétyléniques et/ou des hydrocarbures de type dièniques contenant deux doubles liaisons oléfiniques. Ces composés polyinsaturés sont pratiquement toujours en concentration trop faible pour que l'on puisse envisager de les séparer, par exemple par des méthodes physiques, en vue de leur utilisation pour la pétrochimie. Cependant leur présence, même en quantité ne dépassant pas le plus souvent 1 à 2 % en poids par rapport au poids total de la coupe d'hydrocarbures, à coté des hydrocarbures monooléfiniques rend difficile et même parfois impossible l'utilisation directe de ces coupes dans de nombreux procédés de la pétrochimie. C'est ainsi que dans le procédé d'alkylation des butènes contenus dans les coupes C₄ par l'isobutane en présence d'un catalyseur acide tel que l'acide sulfurique la présence de butadiène entraîne un effet de surconsommation d'acide et de formation de boues acides qu'il faut ensuite éliminer, ce qui pénalise largement le procédé.

Dans le cas de l'hydrogénation sélective des composés polyinsaturés contenus dans les coupes C₂ à C₂₀ et en particulier C₂, C₃, C₄ ou C₅ on constate une dégradation de la sélectivité. Cette dégradation est en particulier très importante dans le cas d'un écoulement des fluides de haut en bas dans un réacteur travaillant à flux descendant. Dans ce cas cette dégradation est d'autant plus importante que la vitesse superficielle de la phase liquide est faible et que celle de la phase gazeuse est forte. Ce problème limite donc largement les possibilités de développement industriel d'un tel réacteur souvent dénommé par les hommes du métier réacteur "trickle bed", car, pour une conversion donnée, si on veut avoir une sélectivité suffisante il faudra pour pouvoir opérer, à des vitesses de circulation de fluides raisonnables, des réacteurs de dimensions très importantes donc relativement chers et difficiles à mettre en oeuvre.

Sans vouloir être lié par une quelconque théorie, il semble que cette dégradation des performances du réacteur soit due, au moins en partie, à des problèmes de distribution de fluides et de ségrégation des phases gazeuse et liquide qui entraîne qu'une partie du liquide se trouve au contact du catalyseur d'hydrogénation dans des conditions telles que la quantité d'hydrogène présente en cet endroit est notablement en dehors du rapport idéal (c'est-à-dire en excès ou en manque) pour assurer une sélectivité et une conversion optimales à cette réaction d'hydrogénation sélective.

Le problème posé est donc de trouver un moyen ou une méthode qui permette d'obtenir pour une conversion donnée de bonnes performances en termes de sélectivité de l'hydrogénation tout en gardant à un niveau le plus faible possible l'hydrogènation et l'isomérisation des oléfines alpha, qui sont le plus souvent celles que l'on souhaite obtenir.

La présente invention propose une solution à ce problème permettant d'améliorer largement la sélectivité de la réaction d'hydrogénation tout en conservant une conversion élevée le plus souvent supérieure à 90 %. Cette solution peut avantageusement être appliquée au cas de coupes C₃ contenant du propadiène et du méthylacétylène, à celui des coupes C₄ contenant du butadiène, à celui des coupes d'essences contenant des composés styrèniques, ou encore à celui des coupes C₈ contenant du phénylacétylène.

Ces coupes C₄ sont par exemple celles que l'on souhaite utiliser pour fabriquer des dimères d'oléfines ou encore que l'on souhaite utiliser pour optimiser la production de butène-1 servant à la fabrication de polymères ou de copolymères et dont l'homme de métier sait que, dans ce cas, ces coupes doivent impérativement contenir une quantité de butadiène la plus faible possible.

La présente invention propose donc un moyen permettant un mélange des phases liquide(s) et gazeuse avant le contact avec le lit de catalyseur (et de préférence avant chaque lit) et permettant une distribution homogène dudit mélange sur toute la section du lit de catalyseur.

La présente invention concerne plus précisèment un procédé d'hydrogénation sélective d'une coupe d'hydrocarbures, ayant 2 à 20 atomes de carbone, renfermant des hydrocarbures oléfiniques monoinsaturés et/ou des aromatiques et au moins un hydrocarbure polyinsaturé appartenant au groupe formé par les composés acétyléniques et les composés diéniques, dans lequel ladite coupe d'hydrocarbures, au moins en partie en phase liquide, et de l'hydrogène circulent selon une direction donnée, dans un réacteur contenant au moins un lit fixe d'un catalyseur d'hydrogénation sous forme de solide divisé, caractérisé en ce que ledit réacteur est muni d'au moins une conduite d'entrée du mélange de fluides comprenant ladite coupe d'hydrocarbures et l'hydrogène et d'au moins une conduite de sortie de la coupe d'hydrocarbures hydrogénés, et en ce qu'il comprend au moins un mélangeur statique en amont de ladite sortie de la coupe d'hydrocarbures hydrogénés.

Le plus souvent le réacteur sera un réacteur allongé le long d'un axe, dont la section, qui peut être quelconque, est le plus souvent carrée, rectangulaire ou circulaire. On utilise habituellement un réacteur de section circulaire et qui comprend outre une conduite d'entrée débouchant dans le réacteur, habituellement de manière à ce que les fluides soient introduits selon la direction de l'axe dudit réacteur, une conduite de sortie habituellement orientée, au moins à proximité immédiate du réacteur, selon l'axe dudit réacteur. Les diamètres de réacteur sont généralement de l'ordre de 0,5 m à 5 m, et de préférence 0,5 à 2,5 m.

Dans le cadre de la présente invention on utilisera l'un des mélangeurs statiques bien connus de l'homme de métier. À titre d'exemple non limitatif de mélangeur statique on peut employer l'un de ceux décrits et vendus par la société SULZER et par exemple ceux connus sous la référence SMV ou SMX décrits en particulier dans la revue Chemical Engineering Progress, Vol. 75, N° 4, avril 1979 pages 61 à 65. Un autre type de mélangeur statique est également décrit dans le brevet EP-B-212 202. On trouvera en outre des descriptions de mélangeur statiques utilisables dans le cadre de la présente invention dans le livre intitulé "Les réacteurs chimiques, conception, calcul et mise en oeuvre" publié par les éditions TECHNIP en 1984 en particulier pages 599 à 605.

Selon une forme particulière de réalisation du procédé selon la présente invention au moins un mélangeur statique sera situé dans la conduite d'entrée dans le réacteur du mélange de fluide comprenant ladite coupe d'hydrocarbures et l'hydrogène.

Selon une autre forme de réalisation au moins un mélangeur statique est situé dans le réacteur en amont du lit de catalyseur, dans le sens de la traversée du réacteur par les fluides, ledit mélangeur assurant en outre une fonction de distribution des fluides.

Selon une autre variante, au moins un mélangeur statique est situé dans le réacteur en amont du lit de catalyseur, dans le sens de la traversée du réacteur par les fluides. Par exemple, le réacteur comporte plusieurs lits et il y a au moins un mélangeur statique entre deux lits successifs.

Dans une réalisation particulière de l'invention le réacteur comprend au moins un mélangeur statique dans lequel est réparti au moins une partie du catalyseur, le reste dudit catalyseur étant au-dessus et/ou en dessous dudit mélangeur statique. Selon cette dernière mise en oeuvre le mélangeur statique pourra dans une version particulière contenir la totalité du catalyseur.

Plusieurs mélangeurs statiques peuvent être employés simultanément et disposés selon l'une des versions de réalisations décrites ci-devant. Lorsque le mélangeur statique ne se trouve pas immédiatement en amont du lit de catalyseur (ou du lit de particules solides non catalytiques si le lit de catalyseur est précédé d'un tel lit de particules solides) on dispose le plus souvent en amont de ce lit un plateau de distribution classique tel que par exemple l'un de ceux décrits à la page 308 ou à la page 490 de l'ouvrage cité ci-devant. Ce plateau de distribution peut être en amont ou en aval d'un mélangeur statique. Lorsque le mélangeur statique se trouve immédiatement en amont du lit de particules solides on utilisera un distributeur de fluide uniquement si ce mélangeur statique n'assure pas en même temps cette fonction de distribution.

Le plus souvent le mélangeur statique sera composé de plaques disposées suivant un certain angle et qui sont empilées de manière à former des canaux ouverts se croisant, disposés obliquement par rapport à l'axe du réacteur.

Selon une forme avantageuse de réalisation de l'invention le réacteur contiendra une pluralité de lits fixes contenant chacun un catalyseur d'hydrogénation, identique ou différent d'un lit à l'autre, séparés les uns des autres par des moyens assurant la collecte des fluides sortant d'un lit fixe de catalyseur, le mélange des fluides ainsi collectés, et la redistribution de ce mélange sur le lit fixe de catalyseur situé en aval dans le sens global de circulation desdits fluides dans ledit réacteur. Dans le cas où le réacteur contient une pluralité de lits catalytiques il est particulièrement avantageux que chaque moyen assurant la collecte des fluides, leur mélange et leur redistribution comporte au moins un moyen d'introduction d'hydrogène gazeux dans le mélange collecté. En restant dans cette forme de réalisation de l'invention le moyen situé entre deux lits de catalyseur et assurant la collecte des fluides, leur mélange et leur redistribution pourra être un mélangeur statique assurant l'ensemble de ces fonctions. On ne sortirait cependant pas du cadre de la présente invention en utilisant un mélangeur statique n'assurant pas la fonction de redistribution des fluides et en lui adjoignant alors un distributeur de fluide classique.

De façon particulièrement préférée selon l'invention, le(s) mélangeur(s) statique(s) situé(s) dans le réacteur occupe(nt) toute la section du réacteur ou plus précisèment, le mélangeur couvre une section au moins égale à celle du lit amont et/ou au moins égale à celle du lit aval (sauf pour le mélangeur situé dans la conduite d'entrée). De cette façon, les vitesses de passage dans le(s) lit(s) et le(s) mélangeur(s) sont sensiblement identiques. Toutes combinaisons des variantes décrites sont possibles.

Le procédé de la présente invention s'applique aussi bien dans le cas où la coupe d'hydrocarbures et l'hydrogène circulent de haut en bas dans le réacteur, que dans le cas où ces fluides circulent de bas en haut. Les vitesses de liquide sont généralement de l'ordre de 0,5 à 8 cm/s, avantageusement 0,5-6 cm/s pour les flux descendants.

Il faut cependant noter que l'amélioration des performances est nettement plus importante dans le cas d'un réacteur à flux descendant qui est ainsi le cas préféré d'application du procédé de la présente invention.

Dans le cadre de la présente invention le catalyseur d'hydrogénation sous forme solide que l'on utilise est un catalyseur classique tel que par exemple l'un de ceux décrits ou cités par Boitiaux et col. dans Hydrocarbon Processing, Mars 1985, pages 51 à 59. L'avantage d'utiliser un catalyseur sous forme de solide divisé en lit fixe (c'est-à-dire avec une répartition aléatoire non organisée) est qu'il apporte un effet de mélange supplémentaire.

Les conditions opératoires applicables
- aux procédés d'hydrogénation des hydrocarbures acétylèniques sont les conditions connues de l'homme de l'art et plus particulièrement une température moyenne comprise entre 10 et 150°C, une pression comprise entre 0,1 et 5MPa et de préférence entre 1,5 et 2,5 MPa et une vitesse spatiale comprise entre 0,5 et 50 volumes de charge liquide par heure et par volume de catalyseur,
- aux procédés d'hydrogénation des hydrocarbures éthylèniques et notamment des diènes contenus dans les charges sont les conditions généralement utilisées pour ce type de transformations et plus particulièrement une température moyenne comprise entre 10 et 100°C, une pression comprise entre 0,1 et 6MPa et de préférence entre 1,5 et 2,5 MPa et une vitesse spatiale comprise entre 0,5 et 6 volumes de charge liquide par heure et par volume de catalyseur,
- aux procédés d'hydrogénation des hydrocarbures aromatiques dans les conditions généralement utilisées pour ce type de transformations et plus particulièrement à une température moyenne comprise entre 50 et 400°C, une pression comprise entre 0,1 et 10MPa et une vitesse spatiale comprise entre 0,5 et 50 volumes de charge liquide par heure et par volume de catalyseur.

Tous les catalyseurs actifs en hydrogénation conviennent.

Le réacteur d'hydrogénation peut fonctionner avec un écoulement de charge sans recyclage de produit ou avec recyclage partiel de produit. Pour des raisons de contrôle de l'exothermicité de la réaction d'hydrogénation, une partie de produit froid est souvent injectée dans l'unité. Ceci est le cas par exemple pour l'hydrogénation des coupes C₃, C₄ et essences de vapocraquage.

Les exemples suivants illustrent l'invention sans en limiter la portée et montre l'amélioration de la sélectivité et de la conversion obtenue en utilisant le procédé de l'invention.

### Exemple 1

On utilise un catalyseur bimétallique comprenant un support d'alumine, en billes, vendu par la société PROCATALYSE sous la référence commerciale LD 271. Le catalyseur est chargé dans un réacteur, de forme allongée le long d'un axe sensiblement vertical, de section sensiblement circulaire de diamètre 10 centimètres. La quantité de catalyseur chargé dans le réacteur est de 21 litres. Avant son utilisation ce catalyseur est réduit par passage d'hydrogène à 150 °C pendant 4 heures. Le lit de catalyseur est surmonté d'un distributeur de fluide. Le réacteur comporte une conduite d'entrée du mélange d'hydrogène et de la coupe C₄ employée dont l'analyse est donnée dans le tableau 1 ci-après. Le mélange de l'hydrogène et de la coupe hydrocarbonée est effectué par introduction de l'hydrogène dans la conduite d'entrée du réacteur. L'hydrogénation sélective du butadiène contenu dans cette coupe d'hydrocarbures est effectuée à flux descendant dans les conditions opératoires suivantes :

| | |
|---|---|
| Pression | 6,5 bars |
| Température | 40 °C |
| Vitesse superficielle du liquide | 1,5 cm/s |
| Rapport molaire Hydrogène/Butadiène initial | 1,2 |

Le produit obtenu après hydrogénation sélective est récupéré en bas du réacteur et une partie du produit est analysée. Les résultats présentés dans le tableau 1 ci-après sont les moyennes des analyses effectuées toutes les deux heures au cours du fonctionnement du réacteur . Le test a duré 200 heures.

**Tableau 1**

| **Composé** | **Charge % poids** | **Produit % poids** |
|---|---|---|
| Isobutane | 26,50 | 26,50 |
| n-Butane | 9,75 | 9,85 |
| Butène-2 cis et trans | 32,00 | 32,45 |
| Butène-1 | 12,70 | 12,79 |
| Isobutène | 14,40 | 14,40 |
| Butadiène | 0,73 | 0,0918 |
| Autres hydrocarbures | 3,92 | 3,92 |

La conversion du butadiène est de 87,47 %. La selectivité (sel1), de la transformation du butadiène en butène-1, égale au rapport du débit molaire du butène-1 formé sur le débit molaire du butadiène global consommé, est de 10,50 %.

### Exemple 2

On utilise le même réacteur que dans l'exemple 1, les mêmes conditions opératoires, et le même catalyseur en quantité identique, mais le réacteur contient deux lits de catalyseur séparés par un mélangeur statique de type SMV vendu par la société SULZER auquel est adjoint un distributeur de liquide immédiatement au dessus de ce mélangeur statique. Le mélangeur statique est formé de trois galettes successives décalées de 90 degrés d'angle l'une par rapport à l'autre comme cela est décrit dans la revue Chemical Engineering Progress, Vol. 75, N° 4, avril 1979 pages 61 à 65. Chaque galette a une hauteur de 10 cm. Le produit obtenu après hydrogénation sélective est récupéré en bas du réacteur et une partie du produit est analysée. Les résultats présentés dans le tableau 2 ci-après sont les moyennes des analyses effectuées toutes les deux heures au cours du fonctionnement du réacteur. Le test a duré 200 heures.

**Tableau 2**

| **Composé** | **Charge % poids** | **Produit % poids** |
|---|---|---|
| Isobutane | 26,50 | 26,50 |
| n-Butane | 9,75 | 9,83 |
| Butène-2 cis et trans | 32,00 | 32,25 |
| Butène-1 | 12,70 | 13,05 |
| Isobutène | 14,40 | 14,40 |
| Butadiène | 0,73 | 0,0468 |
| Autres hydrocarbures | 3,92 | 3,92 |

La conversion du butadiène est de 93,6 %. La selectivité sel1 est égale à 47,56 %.

### Exemple 3

On utilise le même réacteur que dans l'exemple 1, les mêmes conditions opératoires, et le même catalyseur en quantité identique, mais le réacteur contient trois lits de catalyseur séparés chacun par un mélangeur statique de type SMV vendu par la société SULZER auquel est adjoint un distributeur de liquide immédiatement au dessus de ce mélangeur statique. Chaque mélangeur statique est identique à celui employé dans l'exemple 2. Les résultats présentés dans le tableau 3 ci-après sont les moyennes des analyses effectuées toutes les deux heures au cours du fonctionnement du réacteur . Le test a duré 200 heures.

**Tableau 3**

| **Composé** | **Charge % poids** | **Produit % poids** |
|---|---|---|
| Isobutane | 26,50 | 26,50 |
| n-Butane | 9,75 | 9,83 |
| Butène-2 cis et trans | 32,00 | 32,24 |
| Butène-1 | 12,70 | 13,07 |
| Isobutène | 14,40 | 14,40 |
| Butadiène | 0,73 | 0,0439 |
| Autres hydrocarbures | 3,92 | 3,92 |

La conversion du butadiène est de 94,02 %. La selectivité sel1 est égale à 49,57 %.

### Exemple 4

On utilise le même réacteur que dans l'exemple 1, les mêmes conditions opératoires, et le même catalyseur mais en une quantité plus faible. La quantité de catalyseur chargé dans le réacteur est de 15 litres.Le réacteur contient deux lits de catalyseur séparés par un mélangeur statique de type SMV vendu par la société SULZER auquel est adjoint un distributeur de liquide immédiatement au dessus de ce mélangeur statique. Chaque mélangeur statique est identique à celui employé dans l'exemple 2. Les résultats présentés dans le tableau 4 ci-après sont les moyennes des analyses effectuées toutes les deux heures au cours du fonctionnement du réacteur. Le test a duré 200 heures.

**Tableau 4**

| **Composé** | **Charge % poids** | **Produit % poids** |
|---|---|---|
| Isobutane | 26,50 | 26,50 |
| n-Butane | 9,75 | 9,80 |
| Butène-2 cis et trans | 32,00 | 32,19 |
| Butène-1 | 12,70 | 13,10 |
| Isobutène | 14,40 | 14,40 |
| Butadiène | 0,73 | 0,0912 |
| Autres hydrocarbures | 3,92 | 3,92 |

La conversion du butadiène est de 87,55 %. La selectivité sel1 est égale à 57,33 %.

### Exemple 5

On utilise le même réacteur que dans l'exemple 4, les mêmes conditions opératoires, et le même catalyseur en quantité identique, mais le réacteur contient trois lits de catalyseur séparés chacun par un mélangeur statique de type SMV vendu par la société SULZER auquel est adjoint un distributeur de liquide immédiatement au dessus de ce mélangeur statique. Chaque mélangeur statique est identique à celui employé dans l'exemple 2. Les résultats présentés dans le tableau 5 ci-après sont les moyennes des analyses effectuées toutes les deux heures au cours du fonctionnement du réacteur. Le test a duré 200 heures.

**Tableau 5**

| **Composé** | **Charge % poids** | **Produit % poids** |
|---|---|---|
| Isobutane | 26,50 | 26,50 |
| n-Butane | 9,75 | 9,79 |
| Butène-2 cis et trans | 32,00 | 32,20 |
| Butène-1 | 12,70 | 13,10 |
| Isobutène | 14,40 | 14,40 |
| Butadiène | 0,73 | 0,0895 |
| Autres hydrocarbures | 3,92 | 3,92 |

La conversion du butadiène est de 87,79 %. La selectivité sel1 est égale à 57,96 %.

### Exemple 6

On utilise le même réacteur que dans l'exemple 1, les mêmes conditions opératoires, et le même catalyseur en quantité identique, mais la conduite d'entrée du mélange d'hydrogène et de la coupe hydrocarbonée contient un mélangeur statique de type SMV vendu par la société SULZER tel que celui décrit ci-devant dans l'exemple 2 et le réacteur contient entre le distributeur de fluide et le lit de catalyseur un mélangeur statique identique à celui inclus dans la conduite d'entrée. Le produit obtenu après hydrogénation sélective est récupéré en bas du réacteur et une partie du produit est analysée. Les résultats présentés dans le tableau 6 ci-après sont les moyennes des analyses effectuées toutes les deux heures au cours du fonctionnement du réacteur. Le test a duré 200 heures.

**Tableau 6**

| **Composé** | **Charge % poids** | **Produit % poids** |
|---|---|---|
| Isobutane | 26,50 | 26,50 |
| n-Butane | 9,75 | 9,83 |
| Butène-2 cis et trans | 32,00 | 32,24 |
| Butène-1 | 12,70 | 13,07 |
| Isobutène | 14,40 | 14,40 |
| Butadiène | 0,73 | 0,0419 |
| Autres hydrocarbures | 3,92 | 3,92 |

La conversion du butadiène est de 94,29 %. La selectivité sel1 est égale à 51,27 %.

Les exemples ci-devant montrent que le procédé de l'invention permet d'obtenir à isoconversion donnée (exemples 1, 4 et 5 ) une meilleure sélectivité de la conversion de butadiène en butène-1 avec une quantité de catalyseur inférieure. De même les exemples 1, 2 et 3 montrent que pour une quantité de catalyseur donnée la conversion est améliorée et surtout la sélectivité de la conversion de butadiène en butène-1 est très fortement augmentée. La comparaison des résultats obtenus dans l'exemple 1 à ceux obtenus dans l'exemple 6 montre que le procédé de l'invention permet une trés forte augmentation de la sélectivité sel1 et une légère augmentation de la conversion du butadiène.

Le procédé selon l'invention permet donc de
- mélanger les phases liquide(s) et gazeuse entrant dans le réacteur, lorsqu'un mélangeur est situé dans la conduite d'entrée,
- mélanger les phases liquide(s) et gazeuse en avant du lit de façon à assurer un transfert gaz/liquide pour dissoudre l'hydrogène, pour qu'il arrive au contact du solide actif une composition liquide avec un concentration importante au gaz dissous,
- remélanger les phase liquide(s) et gazeuse provenant d'un lit amont, phases qui sont plus ou moins bien séparées et qui peuvent présenter des différences de composition, avant de les mettre en contact avec un lit aval,
- éventuellement de mélanger de l'hydrogène ou du liquide injecté au niveau dudit mélangeur avec les phases liquide(s) et gazeuse présentes dans le réacteur,
- distribuer sur toute la section du lit ledit mélange de phases de façon homogène, les concentrations de liquide et de gaz sont alors réparties uniformément sur toutes la section du lit
- et de façon annexe, d'égaliser les températures sur toute une section.

## Revendications

1. Procédé d'hydrogénation sélective d'une coupe d'hydrocarbures, ayant 2 à 20 atomes de carbone, renfermant des hydrocarbures oléfiniques monoinsaturés et/ou aromatiques et au moins un hydrocarbure polyinsaturé appartenant au groupe formé par les composés acétyléniques et les composés diéniques, dans lequel ladite coupe d'hydrocarbures, au moins en partie en phase liquide, et de l'hydrogène circulent selon une direction donnée, dans un réacteur contenant au moins un lit fixe d'un catalyseur d'hydrogénation sous forme de solide divisé, caractérisé en ce que ledit réacteur est muni d'au moins une conduite d'entrée du mélange de fluides comprenant ladite coupe d'hydrocarbures et l'hydrogène et d'au moins une conduite de sortie de la coupe d'hydrocarbures hydrogénés, et en ce qu'il comprend au moins un mélangeur statique en amont de ladite sortie de la coupe d'hydrocarbures hydrogénés.

2. Procédé selon la revendication 1 dans lequel au moins un mélangeur statique est situé dans la conduite d'entrée dans le réacteur du mélange de fluide comprenant ladite coupe d'hydrocarbures et l'hydrogène.

3. Procédé selon la revendication 1 ou 2 dans lequel au moins un mélangeur statique est situé dans le réacteur en amont au moins d'un lit de catalyseur, dans le sens de la traversée du réacteur par les fluides, ledit mélangeur assurant en outre une fonction de distribution des fluides.

4. Procédé selon l'une des revendications 1 à 3 dans lequel le réacteur comprend au moins un mélangeur statique dans lequel est réparti au moins une partie du catalyseur, le reste dudit catalyseur étant au-dessus et/ou en dessous dudit mélangeur statique.

5. Procédé selon la revendication 4 dans lequel le mélangeur statique contient la totalité du catalyseur.

6. Procédé selon l'une des revendications 1 à 5 dans lequel la coupe d'hydrocarbures et l'hydrogène circulent de haut en bas dans le réacteur.

7. Procédé selon l'une des revendications 1 à 5 dans lequel la coupe d'hydrocarbures et l'hydrogène circulent de bas en haut dans le réacteur.

8. Procédé selon l'une des revendications 1 à 7 dans lequel le mélangeur statique est composé de plaques disposées suivant un certain angle et qui sont empilées de manière à former des canaux ouverts se croisant, disposés obliquement par rapport à l'axe du réacteur.

9. Procédé selon l'une des revendications 1 à 8 dans lequel le réacteur contient une pluralité de lits fixes contenant chacun un catalyseur d'hydrogénation, identique ou différent d'un lit à l'autre, séparés les uns des autres par des moyens assurant la collecte des fluides sortant d'un lit fixe de catalyseur, le mélange des fluides ainsi collectés, et la redistribution de ce mélange sur le lit fixe de catalyseur situé en aval dans le sens global de circulation desdits fluides dans ledit réacteur.

10. Procédé selon la revendication 9 dans lequel chaque moyen assurant la collecte des fluides, leur mélange et leur redistribution comporte au moins un moyen d'introduction d'hydrogène gazeux dans le mélange collecté.

11. Procédé selon la revendication 9 ou 10 dans lequel le moyen situé entre deux lits de catalyseur et assurant la collecte des fluides, leur mélange et leur redistribution est un mélangeur statique.

## Patentansprüche

1. Verfahren zum selektiven Hydrieren einer Kohlenwasserstofffraktion mit 2 bis 20 Kohlenstoffatomen, olefinische monoungesättigte und/oder aromatische Kohlenwasserstoffe und wenigstens einen polyungesättigten Kohlenwasserstoff umfassend, der zu der Gruppe gehört, die gebildet wird durch die acetylenischen Verbindungen und die dienischen Verbindungen, bei dem diese Kohlenwasserstofffraktion, wenigstens zum Teil in flüssiger Phase, und Wasserstoff gemäß einer gegebenen Richtung in einem Reaktor, der wenigstens ein festes Bett aus einem Hydrierungskatalysator in fester unterteilter Form enthält, zirkulieren, dadurch gekennzeichnet, daß dieser Reaktor mit wenigstens einer Eintrittsleitung des Fluidgemisches, diese Gruppe aus Kohlenwasserstoffen und Wasserstoff umfassend sowie einer Austrittsleitung für die Fraktion hydrierter Kohlenwasserstoffe ausgestattet ist und daß er wenigstens einen statischen Mischer in Strömungsrichtung vor dem Ausgang für die Fraktion aus hydrierten Kohlenwasserstoffen umfaßt.

2. Verfahren nach Anspruch 1, bei dem wenigstens ein statischer Mischer in der Eintrittsleitung in den Reaktor für das Fluidgemisch, das wenigstens diese Kohlenwasserstofffraktion sowie Wasserstoff umfaßt, angeordnet ist.

3. Verfahren nach Anspruch 1 oder 2, bei dem wenigstens ein statischer Mischer im Reaktor in Strömungsrichtung vor wenigstens einem Katalysatorbett in Richtung der Durchströmung des Reaktors durch die Fluide angeordnet ist, wobei dieser Mischer im übrigen eine Funktion der Verteilung der Fluide sicherstellt.

4. Verfahren nach einem der Ansprüche 1 bis 3, bei dem der Reaktor wenigstens einen statischen Mischer umfaßt, in dem wenigstens ein Teil des Katalysators verteilt ist, wobei der Rest dieses Katalysators sich oberhalb und/oder unterhalb dieses statischen Mischers befindet.

5. Verfahren nach Anspruch 4, bei dem der statische Mischer die Gesamtheit des Katalysators enthält.

6. Verfahren nach einem der Ansprüche 1 bis 5, bei dem die Kohlenwasserstofffraktion und der Wasserstoff in dem Reaktor von oben nach unten strömen.

7. Verfahren nach einem der Ansprüche 1 bis 5, bei dem die Fraktion aus Kohlenwasserstoffen und der Wasserstoff von unten nach oben im Reaktor strömen.

8. Verfahren nach einem der Ansprüche 1 bis 7, bei dem der statische Mischer gebildet ist aus Platten, die gemäß einem gewissen Winkel angeordnet sind und die derart gestapelt sind, daß sich kreuzende offene Kanäle gebildet werden, welche schräg bezüglich der Achse des Reaktors angeordnet sind.

9. Verfahren nach einem der Ansprüche 1 bis 8, bei dem der Reaktor eine Vielzahl fester Betten enthält, die je einen Hydrierungskatalysator, identisch oder unterschiedlich von einem Bett zum anderen, enthalten, die voneinander durch Mittel getrennt sind, die für das Sammeln der aus einem festen Katalysatorbett austretenden Fluide, das Mischen der so gesammelten Fluide und die Wiederverteilung dieses Gemisches auf das feste Katalysatorbett sicherstellen, das in globaler Strömungsrichtung dieser Fluide in diesem Reaktor abströmseitig angeordnet ist.

10. Verfahren nach Anspruch 9, bei dem jedes für das Sammeln der Fluide, ihr Mischen und ihr Wiederverteilen sorgendes Mittel wenigstens ein Mittel zum Einführen gasförmigen Wasserstoffs in das gesammelte Gemisch umfaßt.

11. Verfahren nach Anspruch 9 oder 10, bei dem das zwischen zwei Katalysatorbetten angeordnete Mittel, das für das Sammeln der Fluide, ihr Mischen und ihre Wiederverteilung sorgt, ein statischer Mischer ist.

## Claims

1. A process for the selective hydrogenation of a fraction of hydrocarbons containing 2 to 20 carbon atoms and comprising monounsaturated olefinic hydrocarbons and/or aromatic compounds and at least one polyunsaturated hydrocarbon from the group formed by acetylenic compounds and dienes, in which the hydrocarbon fraction, which is at least partially in the liquid phase, circulates with hydrogen in a given direction in a reactor containing at least one fixed bed of a hydrogenation catalyst in the form of a divided solid, characterised in that said reactor is provided with at least one inlet conduit for a fluid mixture comprising said hydrocarbon fraction and hydrogen and at least one outlet conduit for the hydrogenated hydrocarbon fraction, and in that it comprises at least one static mixer upstream of said outlet for the hydrogenated hydrocarbon fraction.

2. A process according to claim 1, in which at least one static mixer is located in the inlet conduit into the reactor for mixing the fluid comprising said hydrocarbon cut and hydrogen.

3. A process according to claim 1 or claim 2, in which at least one static mixer is located in the reactor upstream of at least one catalyst bed, in the transit direction of fluid through the reactor, said mixer also acting as a distributor for the fluids.

4. A process according to any one of claims 1 to 3, in which the reactor comprises at least one static mixer in which at least a portion of the catalyst is distributed, the remainder of said catalyst being above and/or below said static mixer.

5. A process according to claim 4, in which the static mixer contains all the catalyst.

6. A process according to any one of claims 1 to 5, in which the hydrocarbon cut circulates with hydrogen from top to bottom in the reactor.

7. A process according to any one of claims 1 to 5, in which the hydrocarbon cut circulates with hydrogen from bottom to top in the reactor.

8. A process according to any one of claims 1 to 7, in which the static mixer is composed of plates located at a certain angle and arranged so as to form open channels which cross each other, located obliquely to the axis of the reactor.

9. A process according to any one of claims 1 to 8, in which the reactor contains a plurality of fixed beds each containing a hydrogenation catalyst, which may be identical or different, separated from each other by means for collecting the fluids leaving a fixed catalyst bed, mixing the collected fluids, and redistributing this mixture over the fixed catalyst bed located downstream in the global direction of circulation of said fluids in the reactor.

10. A process according to claim 9, in which each means for collecting, mixing and redistributing fluids comprises at least one means for introducing hydrogen gas into the collected mixture.

11. A process according to claim 9 or claim 10, in which the means located between two catalyst beds for collecting, mixing and redistributing fluids is a static mixer.
